# EUROPEAN PATENT APPLICATION

(11) **EP 3 875 099 A1**
(43) Date of publication of application: **08.09.2021**
(21) Application number: 19880867.7
(22) Date of filing: 30.09.2019
(51) Int. Cl.: A61K 35/747, A61K 8/9789, A61K 8/99, A61K 36/232, A61K 36/282, A61K 48/00, A61P 43/00, A61Q 19/00, A61Q 19/08, C12N 1/20, C12P 1/04

(54) **HEAT SHOCK PROTEIN GENE EXPRESSION REGULATOR, MEDICINE, COSMETIC, AND METHOD FOR PRODUCING HEAT SHOCK PROTEIN GENE EXPRESSION REGULATOR**

(30) Priority: 30.10.2018 JP 2018203795
(71) Applicant: Murata Manufacturing Co., Ltd., Nagaokakyo-shi, Kyoto 617-8555 (JP)
(72) Inventor: SUNAHARA, Hirofumi, Nagaokakyo-shi, Kyoto 617-8555 (JP); YAMADA, Takaaki, Nagaokakyo-shi, Kyoto 617-8555 (JP)
(74) Representative: Reeve, Nicholas Edward
(86) International application number: PCT/JP2019/038560
(87) International publication number: WO 2020/090321

(57) **Abstract**

An agent for modulating expression of a heat shock protein gene, comprising a *Lactobacillus* sp. derived from *Artemisia indica var. maximowiczii* or *Angelica keiskei.*

## Description

### Technical Field

The present invention relates to an agent for modulating expression of a heat shock protein gene, a medicament, a cosmetic, and a method for manufacturing an agent for modulating expression of a heat shock protein gene.

### Background Art

In recent years, as researches on the structure of human skin and the mechanisms of its metabolism progress, the causes and mechanisms of changes with aging such as wrinkles, fine lines, blemishes, and sagging in human skin have been gradually clarified. The skin is composed of an outer thin epidermis (epithelial tissue) and a thick dermis (connective tissue) in its lower layer. The epidermis, as the outermost layer of the body, protects living body from the outside world and prevents internal moisture and nutrients from leaking to the outside world. The dermis is a connective tissue with a three-dimensionally spreading composite structure mainly composed of fibroblasts, collagen fibers (collagen), elastic fibers (elastin), and proteoglycans, and plays a role in providing strength, stretchability, and elasticity to the skin. As the amount of sebum and moisture in the skin decreases with aging, the moisturizing power of the stratum corneum on the skin surface is lost, and small wrinkles and skin roughness due to dryness or the like tend to occur. Meanwhile, it has been proposed to improve skin function using a fermented product (see, e.g., Patent Literatures 1 to 7). It has also been proposed to induce expression of a heat shock protein and provide a whitening effect by administering an extract of Arnica or the like (see, e.g., Patent Literature 8).

### Citation List

### Patent Literatures

Patent Literature 1: WO 2018/123828
Patent Literature 2: Japanese Patent No. 5468183
Patent Literature 3: Japanese Patent Laid-Open No. 2015-156832
Patent Literature 4: Japanese Patent No. 5467106
Patent Literature 5: Japanese Patent No. 4990297
Patent Literature 6: Japanese Patent Laid-Open No. 2009-249366
Patent Literature 7: Japanese Patent Laid-Open No. 2009-249365
Patent Literature 8: Japanese Patent No. 5697879

### Summary of Invention

### Technical Problem

An object of the present invention is to provide an agent for modulating expression of a heat shock protein gene, a medicament, and a cosmetic, comprising a component derived from a natural source as an active ingredient and having a wide range of effects, and a method for manufacturing an agent for modulating expression of a heat shock protein gene.

### Solution to Problem

The agent for modulating expression of a heat shock protein gene according to one aspect of the present invention comprises a *Lactobacillus* sp. derived from *Artemisia indica var. maximowiczii* or *Angelica keiskei.* The medicament according to one aspect of the present invention comprises a *Lactobacillus* sp. derived from *Artemisia indica var. maximowiczii* or *Angelica keiskei.* The cosmetic according to one aspect of the present invention comprises a *Lactobacillus* sp. derived from *Artemisia indica var. maximowiczii* or *Angelica keiskei.* The method for manufacturing an agent for modulating expression of a heat shock protein gene according to one aspect of the present invention comprises obtaining a *Lactobacillus* sp. from *Artemisia indica var. maximowiczii* or *Angelica keiskei.*

### Advantageous Effects of Invention

According to the present invention, an agent for modulating expression of a heat shock protein gene, a medicament, and a cosmetic, comprising a component derived from a natural source as an active ingredient and having a wide range of effects, and a method for manufacturing an agent for modulating expression of a heat shock protein gene can be provided.

### Brief Description of Drawings

[Figure 1] Figure 1 is a graph and a table showing the analysis results of bacteria contained in a fermentation liquid according to Example 1.
[Figure 2] Figure 2 is a graph and a table showing the analysis results of bacteria contained in a fermentation liquid according to Example 2.
[Figure 3] Figure 3 is a table showing the analysis results of gene expression according to Example 4.
[Figure 4] Figure 4 is a table showing the analysis results of gene expression according to Example 4.
[Figure 5] Figure 5 is a table showing the analysis results of gene expression according to Example 4.
[Figure 6] Figure 6 is a table showing the analysis results of gene expression according to Example 4.
[Figure 7] Figure 7 is a table showing the analysis results of gene expression according to Example 4.
[Figure 8] Figure 8 is a table showing the analysis results of gene expression according to Example 4.
[Figure 9] Figure 9 is a photograph of a human administered a fermentation liquid according to Example 5.
[Figure 10] Figure 10 is a graph showing the anti-bacterial effect of a fermentation liquid according to Example 6.
[Figure 11] Figure 11 is a graph showing the anti-fungal effect of a fermentation liquid according to Example 7.
[Figure 12] Figure 12 is a graph showing the anti-viral effect of a fermentation liquid according to Example 8.
[Figure 13] Figure 13 is a graph showing the anti-viral effect of a fermentation liquid according to Example 8.
[Figure 14] Figure 14 is a graph showing the anti-Trichophyton effect of a fermentation liquid according to Example 9.
[Figure 15] Figure 15 is a table showing the production amount of collagen type I according to Example 10.
[Figure 16] Figure 16 is a table showing the production amount of HSP47 according to Example 10.
[Figure 17] Figure 17 is a micrograph of SA-β-Gal stained cells according to Example 11.
[Figure 18] Figure 18 is a micrograph of SA-β-Gal stained cells according to Example 11.
[Figure 19] Figure 19 is a table showing the degree of SA-β-Gal staining according to Example 11.
[Figure 20] Figure 20 is a table showing the cell viability according to Example 12.
[Figure 21] Figure 21 is a table showing the production amount of HSP70 according to Example 12.

### Description of Embodiments

Hereinafter, embodiments of the present invention are described in detail. It should be noted that the embodiments shown below are examples of apparatuses and methods for embodying the technical ideas of the present invention, and the technical ideas of the present invention are not limited to combinations of components and the like described below. The technical ideas of the present invention can make various changes in the claims.

The agent for modulating expression of a heat shock protein (HSP) gene according to the embodiments comprises a *Lactobacillus* sp. derived from *Artemisia indica var. maximowiczii* (Japanese mugwort) or *Angelica keiskei* (Ashitaba). The agent for modulating expression of a heat shock protein gene according to the embodiments, in particular, promotes expression of a heat shock protein (HSP) 70 gene that improves anti-aging function. The *Lactobacillus* sp. is a species of the genus *Lactobacillus* and is a gram-positive facultative anaerobic bacterium. Bacteria of the genus *Lactobacillus* ferment saccharides to produce lactic acid. The bacteria of the genus *Lactobacillus* reside in the body of an animal including a human. The *Lactobacillus* sp. according to the embodiments is a *Lactobacillus* sp. derived from *Artemisia indica var. maximowiczii* or *Angelica keiskei.*

For example, the *Lactobacillus* sp. according to the embodiments is extracted by fermentation of *Artemisia indica var. maximowiczii* or *Angelica keiskei.* The agent for modulating expression of a heat shock protein gene according to the embodiments may further comprise a fermentation liquid of *Artemisia indica var. maximowiczii* or *Angelica keiskei.*

Examples of the *Lactobacillus* sp. derived from *Artemisia indica var. maximowiczii* includes *L. parafarraginis, L. parabuchneri, L. buchneri,* and *L. harbinensis.* Examples of the *Lactobacillus* sp. derived from *Angelica keiskei* includes *L. vini* and *L. nagelii.* The agent for modulating expression of a heat shock protein gene according to the embodiments may comprise a plurality of species of the *Lactobacillus* genus.

The agent for modulating expression of a heat shock protein gene according to the embodiments promotes expression of the heat shock protein gene. Examples of the heat shock protein gene include an HSPA1A gene encoding HSP70 and an HSPB1 gene encoding HSP27. HSP70 has a whitening promotion function, an anti-blemish function, an anti-wrinkle function, an anti-stress function, an anti-cell death function, an anti-inflammatory function, a cell-protecting function, a gastric mucosal protection function, and an anti-DNA disorder function. HSP27 has anti-stress and wound healing functions. The agent for modulating expression of a heat shock protein gene according to the embodiments comprises a *Lactobacillus* sp. derived from *Angelica keiskei,* and the agent promotes production of HSP47. HSP47 is a molecular chaperone involved in the biosynthesis of collagen. Thus, the agent for modulating expression of a heat shock protein gene according to the embodiments is capable of improving, for example, an anti-stress function, an anti-cell death function, an anti-inflammatory function, a cell-protecting function, a gastric mucosal protection function, an anti-DNA disorder function, and a wound healing function. The agent for modulating expression of a heat shock protein gene according to the embodiments can be used, for example, as a medicament, a cosmetic, and/or a food for at least one application selected from the group consisting of promoting expression of a heat shock protein, improving whitening promotion function, improving anti-blemish function, improving anti-wrinkle function, improving anti-stress function, improving anti-cell death function, improving anti-inflammatory function, improving cell-protecting function, improving gastric mucosal protection function, improving anti-DNA disorder function, and wound healing. It should be noted that the wrinkles include fine wrinkles.

The agent for modulating expression of a heat shock protein gene according to the embodiments also modulates expression of genes other than the heat shock protein gene. For example, the agent for modulating expression of a heat shock protein gene according to the embodiments suppresses expression of an acid ceramide catabolic enzyme (ceramidase) gene. Examples of the acid ceramidase gene include an ASAH1 gene. The agent for modulating expression of a heat shock protein gene according to the embodiments suppresses expression of an acid ceramidase, thus the agent is capable of, for example, promoting ceramide biosynthesis and improving skin barrier function. The agent for modulating expression of a heat shock protein gene according to the embodiments can be used, for example, as a medicament, a cosmetic, and/or a food for at least one application selected from the group consisting of suppressing expression of ceramidase, promoting ceramide biosynthesis, and improving barrier function.

The agent for modulating expression of a heat shock protein gene according to the embodiments promotes expression of a lipid synthase gene. Examples of the lipid synthase gene include a DGAT1 gene. DGAT1 (Diacylglycerol O-acyltransferase 1) synthesizes neutral fat (TAG) from diacylglycerol (DAG). DGAT1-deficient mice exhibit abnormalities in skin barrier function. Thus, the agent for modulating expression of a heat shock protein gene according to the embodiments is capable of, for example, promoting lipid synthesis and improving skin barrier function. The agent for modulating expression of a heat shock protein gene according to the embodiments can be used, for example, as a medicament, a cosmetic, and/or a food for at least one application selected from the group consisting of promoting lipid synthesis and improving barrier function.

The agent for modulating expression of a heat shock protein gene according to the embodiments promotes expression of a lysosomal hydrolase gene. Lysosome is involved in degradation of saccharides and glycolipids in cells. Lysosome requires a lysosomal hydrolase to perform the function. Examples of the lysosomal hydrolase gene include an acid β-glucosidase (GBA) gene. In patients with lysosomal disease, the activity of GBA is reduced or deficient. In addition, activation of GBA improves skin barrier function. Thus, the agent for modulating expression of a heat shock protein gene according to the embodiments is capable of, for example, preventing and treating lysosomal disease, and improving skin barrier function. The agent for modulating expression of a heat shock protein gene according to the embodiments can be used, for example, as a medicament, a cosmetic, and/or a food for at least one application selected from the group consisting of promoting lysosomal hydrolase expression, improving lysosomal function, preventing lysosomal disease, treating lysosomal disease, and improving barrier function.

The agent for modulating expression of a heat shock protein gene according to the embodiments promotes expression of a tight junction biosynthesis factor gene. Examples of the tight junction biosynthesis factor gene include a claudin 1 (CLDN1) gene and an occludin (OCLN) gene. Claudin and occludin are components of the tight junction. Thus, the agent for modulating expression of a heat shock protein gene according to the embodiments is capable of, for example, promoting biosynthesis of tight junction and improving skin barrier function. The agent for modulating expression of a heat shock protein gene according to the embodiments can be used, for example, as a medicament, a cosmetic, and/or a food for at least one application selected from the group consisting of promoting expression of a tight junction biosynthesis factor, promoting expression of claudin, promoting expression of occludin, promoting biosynthesis of tight junction, and improving barrier function.

The agent for modulating expression of a heat shock protein gene according to the embodiments promotes expression of an intercellular adhesion factor gene. Examples of the intercellular adhesion factor gene include an integrin α2 (ITGA2) gene, an E-cadherin (CDH1) gene, and a hyaluronic acid receptor (CD44) gene. Integrin, cadherin, and CD44 contribute to intercellular adhesion. Thus, the agent for modulating expression of a heat shock protein gene according to the embodiments is capable of, for example, promoting biosynthesis of an intercellular adhesion factor and improving skin barrier function. The agent for modulating expression of a heat shock protein gene according to the embodiments can be used, for example, as a medicament, a cosmetic, and/or a food for at least one application selected from the group consisting of promoting biosynthesis of an intercellular adhesion factor, promoting expression of integrin, promoting expression of cadherin, promoting expression of a hyaluronic acid receptor, and improving barrier function.

The agent for modulating expression of a heat shock protein gene according to the embodiments promotes expression of a temperature-sensitive transient receptor potential (TRP) channel gene. Examples of the TRP channel gene include a TRPV3 gene. TRPV3 is involved in proliferation and differentiation of keratinocytes that maintain epidermal barrier function. Thus, the agent for modulating expression of a heat shock protein gene according to the embodiments is capable of, for example, promoting biosynthesis of a keratinocyte and improving skin barrier function. The agent for modulating expression of a heat shock protein gene according to the embodiments can be used, for example, as a medicament, a cosmetic, and/or a food for at least one application selected from the group consisting of promoting expression of a TRP channel, promoting biosynthesis of a keratinocyte, and improving barrier function.

The agent for modulating expression of a heat shock protein gene according to the embodiments promotes expression of an anti-microbial molecule gene. Examples of the anti-microbial molecule gene include a Toll-like receptor 2 (TLR2) gene. Toll-like receptors have functions of sensing microbes and activating immunity. Further examples of the anti-microbial molecule gene include a DEFB1 gene, a DEFB4A gene, and a DEFB103A gene. The DEFB1, DEFB4A, and DEFB103A genes encode β-defensins, such as anti-microbial peptides DEFB1, DEFB2, and DEFB103. Thus, the agent for modulating expression of a heat shock protein gene according to the embodiments is capable of, for example, improving anti-microbial function. The agent for modulating expression of a heat shock protein gene according to the embodiments can be used, for example, as a medicament, a cosmetic, and/or a food for at least one application selected from the group consisting of promoting expression of a Toll-like receptor, promoting expression of a β-defensin, and improving anti-microbial function.

The agent for modulating expression of a heat shock protein gene according to the embodiments promotes expression of a sirtuin gene. Examples of the sirtuin gene include a SIRT4 gene. The activity of the sirtuin gene improves anti-aging function. Thus, the agent for modulating expression of a heat shock protein gene according to the embodiments is capable of, for example, improving anti-aging function. The agent for modulating expression of a heat shock protein gene according to the embodiments can be used, for example, as a medicament, a cosmetic, and/or a food for at least one application selected from the group consisting of promoting expression of sirtuin and improving anti-aging function.

The agent for modulating expression of a heat shock protein gene according to the embodiments promotes expression of a telomerase gene. Examples of the telomerase gene include a TERT gene and a TERC gene. Promoting the activity of telomerases increases cell life. Thus, the agent for modulating expression of a heat shock protein gene according to the embodiments is capable of, for example, improving anti-aging function. The agent for modulating expression of a heat shock protein gene according to the embodiments can be used as a medicament, a cosmetic, and/or a food for at least one application selected from the group consisting of promoting expression of a telomerase and improving anti-aging function.

The agent for modulating expression of a heat shock protein gene according to the embodiments promotes expression of an energy metabolizing factor gene. Examples of the energy metabolizing factor gene include a PPARGC1A gene. The PPARGC1A gene encodes peroxisome proliferator-activated receptor gamma coactivator 1 (PGC-1). PGC-1 promotes energy metabolism. Thus, the agent for modulating expression of a heat shock protein gene according to the embodiments is capable of, for example, improving energy metabolic function. The agent for modulating expression of a heat shock protein gene according to the embodiments can be used as a medicament, a cosmetic, and/or a food for at least one application selected from the group consisting of promoting expression of an energy metabolizing factor, promoting expression of PGC-1, and improving energy metabolic function.

The agent for modulating expression of a heat shock protein gene according to the embodiments promotes expression of a hyaluronic acid biosynthesis factor gene. Examples of the hyaluronic acid biosynthesis factor gene include a hyaluronic acid synthase 1 (HAS1) gene, a hyaluronic acid synthase 2 (HAS2) gene, and a hyaluronic acid synthase 3 (HAS3) gene.

The agent for modulating expression of a heat shock protein gene according to the embodiments suppresses expression of a hyaluronic acid degradation factor gene. Examples of the hyaluronic acid degradation factor gene include a hyaluronic acid degradation enzyme 2 (HYAL2) gene and a cell migration-inducing and hyaluronan-binding protein (CEMIP, KIAA1199) gene.

Thus, the agent for modulating expression of a heat shock protein gene according to the embodiments is capable of, for example, improving hyaluronic acid biosynthesis function. Hyaluronic acid is effective in preventing and treating osteoarthritis, improving moisturizing function, removing sagging, and reducing wrinkles. Thus, the agent for modulating expression of a heat shock protein gene according to the embodiments is capable of, for example, preventing and treating osteoarthritis, improving moisturizing function, and reducing sagging and wrinkles. The agent for modulating expression of a heat shock protein gene according to the embodiments can be used, for example, as a medicament, a cosmetic, and/or a food for at least one application selected from the group consisting of promoting expression of a hyaluronic acid synthase enzyme, suppressing expression of a hyaluronic acid degradation factor, promoting biosynthesis of hyaluronic acid, preventing osteoarthritis, treating osteoarthritis, improving moisturizing function, improving anti-sagging function, and improving anti-wrinkle function.

The agent for modulating expression of a heat shock protein gene according to the embodiments promotes expression of a basal membrane biosynthesis factor gene. Examples of the basal membrane biosynthesis factor gene include a laminin α1 (LAMA1) gene, a laminin α5 (LAMA5) gene, a collagen type IV α1 (COL4A1) gene, and a collagen type VII α1 (COL7A1). The agent for modulating expression of a heat shock protein gene according to the embodiments is capable of, for example, promoting biosynthesis of laminin and collagen and promoting biosynthesis of a basal membrane. The agent for modulating expression of a heat shock protein gene according to the embodiments can be used as a medicament, a cosmetic, and/or a food for at least one application selected from the group consisting of, for example, promoting expression of a basal membrane biosynthesis factor, promoting expression of laminin, promoting expression of collagen, and promoting biosynthesis of a basal membrane.

The agent for modulating expression of a heat shock protein gene according to the embodiments promotes expression of collagen type I. Collagen type I provides elasticity to bone. Collagen type I provides strength to skin. The agent for modulating expression of a heat shock protein gene according to the embodiments can be used as a medicament, a cosmetic, and/or a food for promoting expression of collagen type I.

The agent for modulating expression of a heat shock protein gene according to the embodiments promotes expression of a matrix metalloproteinase inhibitor gene. Examples of the matrix metalloproteinase inhibitor gene include a TIMP1 gene. Matrix metalloproteinase inhibitors inhibit matrix metalloproteinase and suppress degradation of extracellular matrix. Thus, the agent for modulating expression of a heat shock protein gene according to the embodiments is capable of, for example, suppressing degradation of extracellular matrix. The agent for modulating expression of a heat shock protein gene according to the embodiments can be used, for example, as a medicament, a cosmetic, and/or a food for at least one application selected from the group consisting of promoting expression of a matrix metalloproteinase inhibitor and suppressing degradation of extracellular matrix.

The agent for modulating expression of a heat shock protein gene according to the embodiments promotes expression of an anti-oxidation factor gene. Examples of the anti-oxidant factor gene include a superoxide dismutase 3 (SOD3) gene, a catalase (CAT) gene, a glutathione reductase (GSR) gene, a glutathione peroxidase 1 (GPX1) gene, and a metallothionein 1F (MT1F) gene. SOD is an enzyme that degrades active oxygen. CAT is an enzyme that degrades hydrogen peroxide. GSR is an enzyme that reduces oxidative glutathione. GPX1 is an enzyme that degrades hydrogen peroxide. MT1F is an anti-oxidation protein. Thus, the agent for modulating expression of a heat shock protein gene according to the embodiments is capable of, for example, improving anti-oxidation function. The agent for modulating expression of a heat shock protein gene according to the embodiments can be used, for example, as a medicament, a cosmetic, and/or a food for at least one application selected from the group consisting of promoting expression of an anti-oxidation factor gene, promoting expression of SOD, promoting expression of CAT, promoting expression of GSR, promoting expression of GPX1, promoting expression of MT1F, and improving anti-oxidation function.

The agent for modulating expression of a heat shock protein gene according to the embodiments promotes expression of an interleukin 1 receptor antagonist molecule (IL1RN) gene. Deficiency of interleukin-1 receptor antagonist molecules results in deficiency of interleukin-1 receptor antagonist (DIRA). Thus, the agent for modulating expression of a heat shock protein gene according to the embodiments is capable of preventing and treating DIRA. The agent for modulating expression of a heat shock protein gene according to the embodiments can be used, for example, as a medicament, a cosmetic, and/or a food for at least one application selected from the group consisting of promoting expression of an interleukin 1 receptor antagonist, preventing DIRA, and treating DIRA.

The agent for modulating expression of a heat shock protein gene according to the embodiments promotes expression of a semaphorin-in gene. Examples of the semaphorin-in gene include a SEMA3A gene. Semaphorin-in is a repulsive guidance factor that exhibits a repulsive effect on neuroaxis extension. Semaphorin suppresses angiogenesis. Semaphorin controls bone mass. Semaphorin-in suppresses itching. Semaphorin has prophylactic and therapeutic effects on atopic dermatitis. Thus, the agent for modulating expression of a heat shock protein gene according to the embodiments is capable of, for example, repulsively guiding neuroaxis extension, suppressing angiogenesis, controlling bone mass, suppressing itching, and preventing and treating atopic dermatitis. The agent for modulating expression of a heat shock protein gene according to the embodiments can be used, for example, as a medicament, a cosmetic, and/or a food for at least one application selected from the group consisting of promoting expression of semaphorin-in, suppressing extension of neuroaxis, suppressing angiogenesis, controlling bone mass, suppressing itching, preventing atopic dermatitis, and treating atopic dermatitis.

The agent for modulating expression of a heat shock protein gene according to the embodiments promotes expression of a nerve growth factor (NGF) gene and a nerve growth factor receptor (NGFR). Nerve growth factors promote nerve growth and maintenance, promote restoration of cranial nerve function, and are effective in preventing and treating Alzheimer's disease and dementia. Nerve growth factors express effects through neuronal growth factor receptors. Thus, the agent for modulating expression of a heat shock protein gene according to the embodiments is capable of, for example, promoting expression of a nerve growth factor, promoting growth and maintenance of a nerve, promoting restoration of cranial nerve function, and preventing and treating Alzheimer's disease and dementia. The agent for modulating expression of a heat shock protein gene according to the embodiments can be used, for example, as a medicament, a cosmetic, and/or a food for at least one application selected from the group consisting of promoting expression of a nerve growth factor, promoting growth and maintenance of a nerve, promoting restoration of cranial nerve function, preventing Alzheimer's disease, treating Alzheimer's disease, preventing dementia, and treating dementia.

The agent for modulating expression of a heat shock protein gene according to the embodiments promotes expression of a nuclear factor κB (NFκB) inhibitor gene. Examples of the NFκB inhibitor gene include a NFKBIA gene. NFκB inhibitors, such as IκBα, inhibit NFκB activity and prevent and treat cancer. Thus, the agent for modulating expression of a heat shock protein gene according to the embodiments is capable of, for example, preventing and treating cancer. The agent for modulating expression of a heat shock protein gene according to the embodiments can be used, for example, as a medicament, a cosmetic, and/or a food for at least one application selected from the group consisting of promoting expression of an NFκB inhibitor, preventing cancer, and treating cancer.

The agent for modulating expression of a heat shock protein gene according to the embodiments promotes expression of a calpastatin (CAST) gene. The calpastatin is a calpain (CAPN) inhibitory protein. In aged cells, CAPN is activated, and CAST disappears. Thus, the agent for modulating expression of a heat shock protein gene according to the embodiments is capable of, for example, suppressing aging. The agent for modulating expression of a heat shock protein gene according to the embodiments can be used as a medicament, a cosmetic, and/or a food for an application selected from the group consisting of promoting expression of a CAPN inhibitory protein and improving anti-aging function.

The agent for modulating expression of a heat shock protein gene according to the embodiments promotes expression of a citrullinated protein activation factor gene. Examples of the citrullinated protein activator gene include peptidyl arginine deiminase 3 (PAD3). PADs activate citrullinated proteins and advance normal epidermal keratinization. Thus, the agent for modulating expression of a heat shock protein gene according to the embodiments is capable of, for example, advancing normal skin keratinization. The agent for modulating expression of a heat shock protein gene according to the embodiments can be used, for example, as a medicament, a cosmetic, and/or a food for an application selected from the group consisting of promoting expression of a citrullinated protein activator and advancing normal skin keratinization.

The agent for modulating expression of a heat shock protein gene according to the embodiments promotes expression of a transglutaminase gene. Examples of the transglutaminase gene include a TGM1 gene. Transglutaminase increases the physical strength of skin surface and enhances moisturizing function. Thus, the agent for modulating expression of a heat shock protein gene according to the embodiments is capable of, for example, increasing skin surface strength or improving skin moisturizing function. The agent for modulating expression of a heat shock protein gene according to the embodiments can be used, for example, as a medicament, a cosmetic, and/or a food for an application selected from the group consisting of promoting expression of transglutaminase, improving skin surface strength, improving skin firmness, and improving skin moisturizing function.

The agent for modulating expression of a heat shock protein gene according to the embodiments promotes expression of an involucrin (IVL) gene. IVL promotes cornified envelope maturation and improves skin moisturizing function. Thus, the agent for modulating expression of a heat shock protein gene according to the embodiments is capable of, for example, promoting cornified envelope maturation and improving skin moisturizing function. The agent for modulating expression of a heat shock protein gene according to the embodiments can be used, for example, as a medicament, a cosmetic, and/or a food for at least one application selected from the group consisting of promoting expression of IVL, promoting cornified envelope maturation, and improving skin moisturizing function.

The agent for modulating expression of a heat shock protein gene according to the embodiments promotes expression of an aquaporin gene. Examples of the aquaporin gene include an AQP3 gene. Aquaporin is involved in the migration of keratinocytes and improves skin moisturizing function. Thus, the agent for modulating expression of a heat shock protein gene according to the embodiments is capable of, for example, improving keratinocyte migration function and improving skin moisturizing function. The agent for modulating expression of a heat shock protein gene according to the embodiments can be used, for example, as a medicament, a cosmetic, and/or a food for at least one application selected from the group consisting of promoting expression of aquaporin, improving keratinocyte migration function, and improving skin moisturizing function.

The agent for modulating expression of a heat shock protein gene according to the embodiments also has a function of reducing fungus (mold). The agent for modulating expression of a heat shock protein gene according to the embodiments, for example, reduces fungus by 80% or more, 85% or more, 90% or more, or 95% or more within 24 hours. Examples of the fungus include, but are not limited to, Trichophyton, Candida, Cryptococcus, and Aspergillus. The agent for modulating expression of a heat shock protein gene according to the embodiments also has a therapeutic effect on mycosis. Examples of the mycosis include, but are not limited to, trichophytosis, candidiasis, cryptococcosis, and aspergillosis.

The agent for modulating expression of a heat shock protein gene according to the embodiments also has a function of reducing gram-negative bacterium and gram-positive bacterium. The agent for modulating expression of a heat shock protein gene according to the embodiments, for example, reduces gram-negative bacterium and gram-positive bacterium by 80% or more, 85% or more, 90% or more, or 95% or more within 24 hours. Examples of the gram-negative bacterium include, but are not limited to, Escherichia coli, Salmonella enterica, Vibrio enteritidis, Bacillus pneumoniae, and Pseudomonas aeruginosa. Examples of the gram-positive bacterium include, but are not limited to, methicillin-resistant Staphylococcus aureus (MRSA), spore-forming Bacillus cereus, and Bacillus subtilis.

The agent for modulating expression of a heat shock protein gene according to the embodiments also has a function of reducing virus. The agent for modulating expression of a heat shock protein gene according to the embodiments, for example, reduces virus by 80% or more, 85% or more, 90% or more, or 95% or more within 24 hours. The virus includes an envelope virus, which is a virus with an envelope, and a non-envelope virus, which is a virus without an envelope. The virus also includes a DNA virus and an RNA virus.

Examples of the DNA virus with an envelope include, but are not limited to, human herpes virus, vaccinia virus, and hepatitis B virus.

Examples of the RNA virus with an envelope include, but are not limited to, influenza virus, SARS coronavirus, RS virus, mumps virus, Lassa virus, dengue virus, rubella virus, human immunodeficiency virus, measles virus, hepatitis C virus, Ebola virus, yellow fever virus, and Japanese encephalitis virus.

Examples of the DNA virus without an envelope include, but are not limited to, adenovirus, B19 virus, papova virus, and human papilloma virus.

Examples of the RNA virus without an envelope include, but are not limited to, norovirus, polio virus, echovirus, hepatitis A virus, hepatitis E virus, rhinovirus, astrovirus, rotavirus, coxsackievirus, enterovirus, and sapovirus.

The agent for modulating expression of a heat shock protein gene according to the embodiments contains an effective amount of a *Lactobacillus* sp. The agent for modulating expression of a heat shock protein gene according to the embodiments may contain the *Lactobacillus* sp. in a solvent such as water or in a fermentation liquid of a plant. Examples of the plant for obtaining the fermentation liquid include *Artemisia indica var. maximowiczii* or *Angelica keiskei,* as in the *Lactobacillus* sp.

The effective amount is an amount necessary to exhibit a gene expression modulation effect. The effective amount is appropriately determined depending on a gene of interest. Generally, as the concentration of the *Lactobacillus* sp. increases and a solvent contains plant fermentation liquid, more effect for modulating expression of a gene is exhibited. However, for example, in the case where the gene of interest is a GBA gene, the lower the concentration of the *Lactobacillus* sp., more effect for modulating expression of a gene is exhibited. Furthermore, for example, in the case where the gene of interest is a TLR2 gene, a solvent containing no plant fermentation liquid leads to more effect for modulating expression of a gene.

The *Lactobacillus* sp. contained in the agent for modulating expression of a heat shock protein gene according to the embodiments may be a live bacterium, or may be a dead bacterium, for example, a heat-treated bacterium. Thus, the agent for modulating expression of a heat shock protein gene according to the embodiments may contain a dead bacterium of the *Lactobacillus* sp. The *Lactobacillus* sp. may be a dried bacterial product. The dead bacterium or dried bacterial product of the *Lactobacillus* sp. also has a gene expression modulation effect and an anti-bacterial anti-viral effect. The dead bacterium or dried bacterial product of the L *Lactobacillus* sp. is easy to transport and store for a long time.

The agent for modulating expression of a heat shock protein gene according to the embodiments can be, for example, a liquid, a cream, an ointment, a plaster, a gel, a wax, or a spray.

The agent for modulating expression of a heat shock protein gene according to the embodiments can be, for example, a cosmetic for skin conditioning. Examples of the cosmetic for skin conditioning include a lotion, an essence, and a pack. The agent for modulating expression of a heat shock protein gene according to the embodiments can be, for example, a cosmetic for protection. Examples of the cosmetic for protection include an emulsion for protection and a cream for protection. The agent for modulating expression of a heat shock protein gene according to the embodiments can be, for example, a base makeup cosmetic. Examples of the base makeup cosmetic include a foundation, a powder, and a foundation primer. The agent for modulating expression of a heat shock protein gene according to the embodiments can be, for example, a point makeup cosmetic. Examples of the point makeup cosmetic include a lipstick, an eye makeup, a cheek, and a nail enamel.

The agent for modulating expression of a heat shock protein gene according to the embodiments is provided as, for example, a disinfectant, a dermatological agent such as a therapeutic ointment agent, an eye drop, or an oral medicine. The agent for modulating expression of a heat shock protein gene according to the embodiments is administered to, for example, skin of a human body including a finger, a toe, a hand, and a foot, a hair, an oral cavity, and an eye.

The agent for modulating expression of a heat shock protein gene can contain, in addition to the *Lactobacillus* sp., a formulation component of cosmetic and medicament, such as a liquid fat, a solid fat, a wax, a hydrocarbon, a higher fatty acid, a higher alcohol, an ester, a silicone, an anionic surfactant, a cationic surfactant, an amphoteric surfactant, a non-ionic surfactant, a moisturizing agent, a water-soluble polymer, a thickening agent, a coating agent, a metal ion blocking agent, a lower alcohol, a polyhydric alcohol, a saccharide, an amino acid, an organic amine, a pH adjusting agent, a skin nutrient, a vitamin, an anti-oxidant, a fragrance, a powder, a coloring material, and water, depending on the purpose, as desired.

In the case where the agent for modulating expression of a heat shock protein gene according to the embodiments contains an oily component, the concentration of the oily component in the agent for modulating expression of a heat shock protein gene according to the embodiments is not particularly limited, but, for example, is 0.1% by mass or more and 90% by mass or less, or 0.5% by mass or more and 90% by mass or less. In the case where the agent for modulating expression of a heat shock protein gene according to the embodiments contains an aqueous component, the concentration of the aqueous component in the agent for modulating expression of a heat shock protein gene according to the embodiments is not particularly limited, but, for example, is 0.1% by mass or more and 90% by mass or less, or alternatively 0.5% by mass or more and 90% by mass or less. The ratio of the oily component to the aqueous component in the agent for modulating expression of a heat shock protein gene according to the embodiments is appropriately set depending on whether the agent for modulating expression of a heat shock protein gene according to the embodiments is an oil-in-water (O/W) agent or a water-in-oil (W/O) agent. In the case where the agent for modulating expression of a heat shock protein gene according to the embodiments contains a surfactant, the concentration of the surfactant in the agent for modulating expression of a heat shock protein gene according to the embodiments is not particularly limited, and, for example, is 2% by mass or more and 10% by mass or less.

The agent for modulating expression of a heat shock protein gene according to the embodiments may contain, as appropriate, an anti-bacterial substance or anti-viral substance according to the purpose, in addition to the *Lactobacillus* sp.

The agent for modulating expression of a heat shock protein gene according to the embodiments is produced by fermenting a plant to obtain a fermentation liquid containing the *Lactobacillus* sp. When the plant is fermented, salt and saccharides such as molasses are added to the plant. The fermentation temperature is, for example, 30°C. The hydrogen ion index (pH) of the resulting fermentation liquid is around 4.0. Secretions of the *Lactobacillus* sp. may be extracted from the fermentation liquid.

The obtained fermentation liquid may be heated to make the *Lactobacillus* sp. contained in the fermentation liquid into a dead bacterium. The fermentation liquid may also be spray-dried to obtain a dried bacterial product of the *Lactobacillus* sp. The dried bacterial product can also be prepared by a lyophilization method, a hot-air drying method, or the like.

Furthermore, the obtained fermentation liquid, a bacterial cell of the *Lactobacillus* sp., or a dried bacterial product of the *Lactobacillus* sp. may be added to soy milk, and the soy milk may be fermented to obtain a soy milk fermentation liquid. The soy milk fermentation liquid also has a gene expression modulation effect.

As described above, the agent for modulating expression of a heat shock protein gene, or the like, according to each embodiment of the present invention has the configuration and the action and effect as shown by the following examples according to any one of the described above or a combination of those.

The agent for modulating expression of a heat shock protein gene, the medicament, or the cosmetic according to the present embodiments contains a *Lactobacillus* sp. derived from *Artemisia indica var. maximowiczii* or *Angelica keiskei.* The agent for modulating expression of a heat shock protein gene, the medicament, or the cosmetic according to the present embodiments can further contain a fermentation liquid derived from *Artemisia indica var. maximowiczii* or *Angelica keiskei.* The medicament may be an anti-wrinkle medicament or an anti-aging medicament. The cosmetic may be an anti-wrinkle cosmetic or an anti-aging cosmetic.

The present embodiments include a *Lactobacillus* sp. derived from *Artemisia indica var. maximowiczii* or *Angelica keiskei,* which is used for modulating expression of a heat shock protein gene. The present embodiments include a *Lactobacillus* sp. derived from *Artemisia indica var. maximowiczii* or *Angelica keiskei* and a fermentation liquid derived from *Artemisia indica var. maximowiczii* or *Angelica keiskei,* which are used for modulating expression of a heat shock protein gene.

The present embodiments include use of a *Lactobacillus* sp. derived from *Artemisia indica var. maximowiczii* or *Angelica keiskei,* for manufacturing an agent for modulating expression of a heat shock protein gene, a medicament, or a cosmetic. The present embodiments include use of a *Lactobacillus* sp. derived from *Artemisia indica var. maximowiczii* or *Angelica keiskei* and a fermentation liquid derived from *Artemisia indica var. maximowiczii* or *Angelica keiskei,* for manufacturing an agent for modulating expression of a heat shock protein gene, a medicament, or a cosmetic.

The present embodiments include a method for modulating expression of a heat shock protein gene, a treatment method, or a cosmetic method, comprising administering to a human or a non-human animal a *Lactobacillus* sp. derived from *Artemisia indica var. maximowiczii* or *Angelica keiskei.* The present embodiments include a method for modulating expression of a heat shock protein gene, a treatment method, or a cosmetic method, comprising administering to a human or a non-human animal a *Lactobacillus* sp. derived from *Artemisia indica var. maximowiczii* or *Angelica keiskei* and a fermentation liquid derived from *Artemisia indica var. maximowiczii* or *Angelica keiskei.* The treatment method may be a treatment method for improving anti-wrinkle function or a treatment method for improving anti-aging function. The cosmetic method may be a cosmetic method for improving anti-wrinkle function or a cosmetic method for improving anti-aging function.

The heat shock protein gene can be at least one selected from the group consisting of an HSPA1A gene and an HSPB1 gene. The *Lactobacillus* sp. derived from *Artemisia indica var. maximowiczii* or *Angelica keiskei* according to the present embodiments may promote expression of at least one selected from the group consisting of the HSPA1A gene and the HSPB1 gene.

The *Lactobacillus* sp. derived from *Artemisia indica var. maximowiczii* or *Angelica keiskei* according to the present embodiments can modulate expression of a gene other than a heat shock protein gene. The gene of which expression is modulated by the *Lactobacillus* sp. derived from *Artemisia indica var. maximowiczii* or *Angelica keiskei* may be at least one selected from the group consisting of a ceramidase gene, a lipid synthase gene, a lysosomal hydrolase gene, a tight junction biosynthesis factor gene, and an intercellular adhesion factor gene.

The ceramidase gene may be an ASAH1 gene. The *Lactobacillus* sp. derived from *Artemisia indica var. maximowiczii* or *Angelica keiskei* according to the present embodiments may suppress expression of the ASAH1 gene.

The lipid synthase gene may be a DGAT1 gene. The *Lactobacillus* sp. derived from *Artemisia indica var. maximowiczii* or *Angelica keiskei* according to the present embodiments may promote expression of the DGAT1 gene.

The lysosomal hydrolase gene may be a GBA gene. The *Lactobacillus* sp. derived from *Artemisia indica var. maximowiczii* or *Angelica keiskei* according to the present embodiments may promote expression of the GBA gene.

The tight junction biosynthesis factor gene may be at least one selected from the group consisting of a CLDN1 gene and an OCLN gene. The *Lactobacillus* sp. derived from *Artemisia indica var. maximowiczii* or *Angelica keiskei* according to the present embodiments may promote expression of at least one selected from the group consisting of the CLDN1 gene and the OCLN gene.

The intercellular adhesion factor gene may be at least one selected from the group consisting of an ITGA2 gene, a CDH1 gene, and a CD44 gene. The *Lactobacillus* sp. derived from *Artemisia indica var. maximowiczii* or *Angelica keiskei* may promote expression of at least one selected from the group consisting of the ITGA2 gene, the CDH1 gene, and the CD44 gene.

The gene of which expression is modulated by the *Lactobacillus* sp. derived from *Artemisia indica var. maximowiczii* or *Angelica keiskei* according to the present embodiments may be an anti-microbial molecule gene. The anti-microbial molecule gene may be at least one selected from the group consisting of a TLR2 gene, a DEFB1 gene, a DEFB4A gene, and a DEFB103A gene. The *Lactobacillus* sp. derived from *Artemisia indica var. maximowiczii* or *Angelica keiskei* according to the present embodiments may promote expression of at least one selected from the group consisting of the TLR2 gene, the DEFB1 gene, the DEFB4A gene, and the DEFB103A gene.

The gene of which expression is modulated by the *Lactobacillus* sp. derived from *Artemisia indica var. maximowiczii* or *Angelica keiskei* according to the present embodiments may be at least one selected from the group consisting of a sirtuin gene and a telomerase gene.

The sirtuin gene may be a SIRT4 gene. The *Lactobacillus* sp. derived from *Artemisia indica var. maximowiczii* or *Angelica keiskei* according to the present embodiments may promote expression of the SIRT4 gene.

The telomerase gene may be at least one selected from the group consisting of a TERT gene and a TERC gene. The *Lactobacillus* sp. derived from *Artemisia indica var. maximowiczii* or *Angelica keiskei* according to the present embodiments may promote expression of at least one selected from the group consisting of the TERT gene and the TERC gene.

The gene of which expression is modulated by the *Lactobacillus* sp. derived from *Artemisia indica var. maximowiczii* or *Angelica keiskei* according to the present embodiments may be an energy metabolizing factor gene. The energy metabolizing factor gene may be a PPARGC1A gene. The *Lactobacillus* sp. derived from *Artemisia indica var. maximowiczii* or *Angelica keiskei* according to the present embodiments may promote expression of the PPARGC1A gene.

The gene of which expression is modulated by the *Lactobacillus* sp. derived from *Artemisia indica var. maximowiczii* or *Angelica keiskei* according to the present embodiments may be at least one selected from the group consisting of a hyaluronic acid biosynthesis factor gene and a hyaluronic acid degradation factor gene.

The hyaluronic acid biosynthesis factor gene may be at least one selected from the group consisting of a HAS1 gene, a HAS2 gene, and a HAS3 gene. The *Lactobacillus* sp. derived from *Artemisia indica var. maximowiczii* or *Angelica keiskei* according to the present embodiments may promote expression of at least one selected from the group consisting of the HAS1 gene, the HAS2 gene, and the HAS3 gene.

The hyaluronic acid degradation factor gene may be at least one selected from the group consisting of a HYAL2 gene and a CEMIP gene. The *Lactobacillus* sp. derived from *Artemisia indica var. maximowiczii* or *Angelica keiskei* according to the present embodiments may suppress expression of at least one selected from the group consisting of the HYAL2 gene and the CEMIP gene.

The gene of which expression is modulated by the *Lactobacillus* sp. derived from *Artemisia indica var. maximowiczii* or *Angelica keiskei* according to the present embodiments may be a basal membrane biosynthesis factor gene. The basal membrane biosynthesis factor gene may be at least one selected from the group consisting of a LAMA1 gene, a LAMA5 gene, a COL4A1 gene, and a COL7A1 gene. The *Lactobacillus* sp. derived from *Artemisia indica var. maximowiczii* or *Angelica keiskei* according to the present embodiments may promote expression of at least one selected from the group consisting of the LAMA1 gene, the LAMA5 gene, the COL4A1 gene, and the COL7A1 gene.

The agent for modulating expression of a heat shock protein gene, the medicament, or the cosmetic according to the present embodiments promotes production of collagen type I. The agent for promoting production of collagen type I according to the present embodiments contains a *Lactobacillus* sp. derived from *Artemisia indica var. maximowiczii* or *Angelica keiskei.* The agent for promoting production of collagen type I according to the present embodiments contains a *Lactobacillus* sp. derived from *Artemisia indica var. maximowiczii* or *Angelica keiskei* and a fermentation liquid derived from *Artemisia indica var. maximowiczii* or *Angelica keiskei.*

The present embodiments include a *Lactobacillus* sp. derived from *Artemisia indica var. maximowiczii* or *Angelica keiskei,* which is used for promoting production of collagen type I. The present embodiments include a *Lactobacillus* sp. derived from *Artemisia indica var. maximowiczii* or *Angelica keiskei* and a fermentation liquid derived from *Artemisia indica var. maximowiczii* or *Angelica keiskei,* which are used for promoting production of collagen type I.

The present embodiments include use of a *Lactobacillus* sp. derived from *Artemisia indica var. maximowiczii* or *Angelica keiskei* for manufacturing an agent for promoting production of collagen type I. The present embodiments include use of a *Lactobacillus* sp. derived from *Artemisia indica var. maximowiczii* or *Angelica keiskei* and a fermentation liquid derived from *Artemisia indica var. maximowiczii* or *Angelica keiskei* for manufacturing an agent for promoting production of collagen type I.

The present embodiments include a method for promoting production of collagen type I, comprising administering a *Lactobacillus* sp. derived from *Artemisia indica var. maximowiczii* or *Angelica keiskei* to a human or a non-human animal. The present embodiments include a method for promoting production of collagen type I, comprising administering a *Lactobacillus* sp. derived from *Artemisia indica var. maximowiczii* or *Angelica keiskei* and a fermentation liquid derived from *Artemisia indica var. maximowiczii* or *Angelica keiskei* to a human or a non-human animal.

The agent for modulating expression of a heat shock protein gene, the medicament, or the cosmetic according to the present embodiments promotes production of HSP47. The agent for promoting production of HSP47 according to the present embodiments contains a *Lactobacillus* sp. derived from *Angelica keiskei.* The agent for promoting production of HSP47 according to the present embodiments contains a *Lactobacillus* sp. derived from *Angelica keiskei* and a fermentation liquid derived from *Angelica keiskei.*

The present embodiments include a *Lactobacillus* sp. derived from *Angelica keiskei,* which is used for promoting production of HSP47. The present embodiments include a *Lactobacillus* sp. derived from *Angelica keiskei* and a fermentation liquid derived from *Angelica keiskei,* which are used for promoting production of HSP47.

The present embodiments include use of a *Lactobacillus* sp. derived from *Angelica keiskei,* for manufacturing an agent for promoting production of HSP47. The present embodiments include use of a *Lactobacillus* sp. derived from *Angelica keiskei* and a fermentation liquid derived from *Angelica keiskei,* for manufacturing an agent for promoting production of HSP47.

The present embodiments include a method for promoting production of HSP47, comprising administering a *Lactobacillus* sp. derived from *Angelica keiskei* to a human or a non-human animal. The present embodiments include a method for promoting production of HSP47, comprising administering a *Lactobacillus* sp. derived from *Angelica keiskei* and a fermentation liquid derived from *Angelica keiskei* to a human or a non-human animal.

The *Lactobacillus* sp. derived from *Artemisia indica var. maximowiczii* or *Angelica keiskei* according to the present embodiments may be at least one selected from the group consisting of *L. parafarraginis, L.* parabuchneri, *L.* buchneri, *L.* harbinensis, *L.* vini, and *L.* nagelii.

The *Lactobacillus* sp. derived from *Artemisia indica var. maximowiczii* or *Angelica keiskei* according to the present embodiments may be a dead bacterium. The *Lactobacillus* sp. derived from *Artemisia indica var. maximowiczii* or *Angelica keiskei* according to the present embodiments may be subjected to a heat treatment. The *Lactobacillus* sp. derived from *Artemisia indica var. maximowiczii* or *Angelica keiskei* according to the present embodiments may be a dried bacterial product.

The method for manufacturing an agent for modulating expression of a heat shock protein gene, a medicament, or a cosmetic according to the present embodiments comprises obtaining a *Lactobacillus* sp. from *Artemisia indica var. maximowiczii* or *Angelica keiskei.* The method for manufacturing an agent for modulating expression of a heat shock protein gene, a medicament, or a cosmetic according to the present embodiments may further comprise fermenting *Artemisia indica var. maximowiczii* or *Angelica keiskei* to obtain a fermentation liquid. The method for manufacturing an agent for modulating expression of a heat shock protein gene, a medicament, or a cosmetic according to the present embodiments may further comprise making the obtained *Lactobacillus* sp. into a dead bacterium. The method for manufacturing an agent for modulating expression of a heat shock protein gene, a medicament, or a cosmetic according to the present embodiments may further comprise subjecting the obtained *Lactobacillus* sp. to a heat treatment. The method for manufacturing an agent for modulating expression of a heat shock protein gene, a medicament, or a cosmetic according to the present embodiments may further comprise drying the obtained *Lactobacillus* sp.

### Examples

Hereinafter, Examples of the present invention are described. However, it must be expected that the present invention is not limited to the following Examples.

### (Example 1: Lactobacillus sp. derived from Artemisia indica var. maximowiczii)

In the leaves of *Artemisia indica var. maximowiczii,* it is supposed that the number of lactic acid bacteria maximizes during a total of 2 hours (before 1 hour from the sunrise time and after 1 hour from the sunrise time) in a day. In addition, it is supposed that the lactic acid bacteria decrease and the photosynthetic bacteria increase outside of the time period. Thus, during the two hours, the portions about 20 cm from the tips of the leaves of *Artemisia indica var. maximowiczii* were harvested. 6.3 kg of the harvested leaves of *Artemisia indica var. maximowiczii* were immediately placed in a first pickle barrel with a plastic bag placed inside. 3.2 kg of molasses and 0.6 kg of coarse salt were sprinkled into the leaves of *Artemisia indica var. maximowiczii,* and then the opening of the plastic bag was closed and sealed. A heavy stone was placed on the top of the plastic bag, and the leaves of *Artemisia indica var. maximowiczii* were pickled.

A several days after the pickle juice had risen to the top of the leaves of *Artemisia indica var. maximowiczii,* the heavy stone was removed. Then, 10 L of water without chlorine for rinsing out was added to a second pickle barrel, and the pickle of the leaves of *Artemisia indica var. maximowiczii* and 10 kg of the pickle juice were added into the water. In addition, a third pickle barrel was prepared, and a wire net filter was placed on the opening of the third pickle barrel. From the second pickle barrel, the leaves of *Artemisia indica var. maximowiczii* were removed little by little while rubbing and washing them by hands, and the leaves of *Artemisia indica var. maximowiczii* were gently pressed by the palm of the hand against the wire net filter on the opening of the third pickle barrel to squeeze the pickle juice.

After squeezing all of the leaves of *Artemisia indica var. maximowiczii,* the pickle juice remaining in the second pickle juice was filtered through the wire net filter. Next, into the pickle juice in the third pickle barrel, molasses (Hateruma brown sugar) was melted so that the final concentration was 10% by weight, and coarse salt was melted so that the final concentration was 3% by weight. The fermentation was then started by setting the ambient temperature of the third pickle barrel to about 30°C. Foaming with large foams was first confirmed, then the foaming was gradually turned into foaming with fine foams, and finally the foaming was ceased. The pH when the foaming was ceased after about a week was around 3.8. The pickle juice at this time was used as a fermentation liquid of *Artemisia indica var. maximowiczii.* A portion of the obtained fermentation liquid of *Artemisia indica var. maximowiczii* was heated at 70°C for 30 minutes to obtain a heat-treated fermentation liquid of *Artemisia indica var. maximowiczii* in which the bacteria were dead.

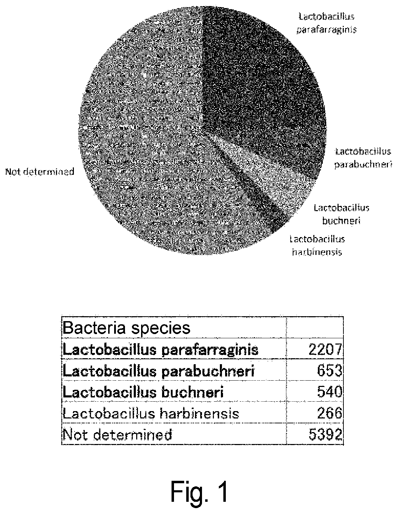

Analysis of the non-heat-treated fermentation liquid of *Artemisia indica var. maximowiczii* by a next-generation sequencer (MiSeq, Illumina, Inc.) showed, as shown in Figure 1, that the fermentation liquid of *Artemisia indica var. maximowiczii* contained a parafarraginis species, a parabuchneri species, a buchneri species, a harbinensis species and the like of the *Lactobacillus* sp. Note that the next-generation sequencer is also referred to as a high-throughput sequencer. The numerical values in the table in Figure 1 reflect the bacterial number of bacterial species contained in the fermentation liquid of *Artemisia indica var. maximowiczii.*

### (Example 2: Lactobacillus sp. derived from Angelica keiskei)

In the leaves of *Angelica keiskei,* it is supposed that the number of lactic acid bacteria maximizes during a total of 2 hours before and after 1 hour from the sunrise time in a day. In addition, it is supposed that, the lactic acid bacteria decrease and the photosynthetic bacteria increase outside of the time period. Thus, during the two hours, the leaf stems of the sprouts of *Angelica keiskei* were harvested. 6.3 kg of the harvested *Angelica keiskei* were immediately placed in a first pickle barrel with a plastic bag placed inside. 3.2 kg of molasses and 0.6 kg of coarse salt were sprinkled into the *Angelica keiskei,* and then the opening of the plastic bag was closed and sealed. A heavy stone was placed on the top of the plastic bag and the *Angelica keiskei* was pickled.

A several days after the pickle juice had risen to the top of the *Angelica keiskei,* the heavy stone was removed. Then, 10 L of water without chlorine for rinsing out was added to a second pickle barrel, and the pickle of the *Angelica keiskei* and 10 kg of the pickle juice were added into the water. In addition, a third pickle barrel was prepared, and a wire net filter was placed on the opening of the third pickle barrel. From the second pickle barrel, the *Angelica keiskei* was removed little by little while rubbing and washing it by hands, and the *Angelica keiskei* was gently pressed by the palm of the hand against the wire net filter on the opening of the third pickle barrel to squeeze the pickle juice.

After squeezing all of the *Angelica keiskei,* the pickle juice remaining in the second pickle juice was filtered through the wire net filter. Next, into the pickle juice in the third pickle barrel, molasses was melted so that the final concentration was 10% by weight, and coarse salt was melted so that the final concentration was 3% by weight. The fermentation was then started by setting the ambient temperature of the third pickle barrel to about 30°C. Foaming with large foams was first confirmed, then the foaming was gradually turned into foaming with fine foams, and finally the foaming was ceased. The pH when the foaming was ceased after about a week was around 4.0. The pickle juice at this time was used as a fermentation liquid of *Angelica keiskei.* A portion of the obtained fermentation liquid of *Angelica keiskei* was heated at 70°C for 30 minutes to obtain a heat-treated fermentation liquid of *Angelica keiskei* in which the bacteria were dead.

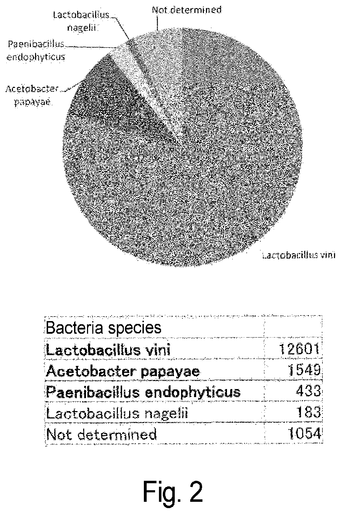

Analysis of the non-heat-treated fermentation liquid of *Angelica keiskei* by a next-generation sequencer (MiSeq, Illumina, Inc.) showed that the fermentation liquid of *Angelica keiskei* contained a vini species, a nagelii species and the like, as shown in Figure 2. The numerical values in the table in Figure 2 reflect the bacterial number of each bacterial species contained in the fermentation liquid of *Angelica keiskei.*

### (Example 3: Soy milk fermentation liquid using Lactobacillus sp.)

Soy milk was heated to 70°C, and superheated and sterilized for about 30 minutes. To the heat-sterilized treated soy milk, the non-heat-treated fermentation liquid of *Artemisia indica var. maximowiczii* prepared in Example 1 was added such that the final concentration was about 10% by weight, and the mixture was sufficiently stirred. Subsequently, the soy milk to which the non-heat-treated fermentation liquid of *Artemisia indica var. maximowiczii* was added was fermented at 37°C for 24 hours. After fermentation, the solids were removed by filtration to obtain a soy milk fermentation liquid containing a *Lactobacillus* sp.

### (Example 4: Gene Expression Test)

A live *Lactobacillus* sp. was isolated from the fermentation liquid of *Artemisia indica var. maximowiczii* obtained in Example 1, then dispersed in pure water at a concentration of 0.05 g/L, and the obtained dispersion was taken as Sample 1. The isolated live *Lactobacillus* sp. was dispersed in pure water at a concentration of 5.0 g/L, and the obtained dispersion was taken as Sample 2. A fermentation liquid of *Artemisia indica var. maximowiczii* containing the live *Lactobacillus* sp. at a concentration of 5.0 g/L was taken as Sample 3.

A three-dimensional culture epidermal model (SkinEtchic RHE: 18 RHE 098, EPISKIN, Inc.) was conditioned overnight using a growth medium (Growth Medium: 18 SGM 082, EPISKIN, Inc.). The three-dimensional culture epidermal model was then transferred to a transwell insert (Corning) of a six-well plate dispensed with the growth medium, and 50 µL of one of Samples 1 to 3 was applied to the stratum corneum side of the three-dimensional culture epidermal model in the well. After 24 hours, the medium to which the sample was added was removed from the well, and the three-dimensional culture epidermal model was washed with phosphate buffered saline (PBS(-)) free of calcium and magnesium.

The three-dimensional culture epidermal model was cut together with the membrane from the transwell insert using a scalpel, and the three-dimensional culture epidermal model was immersed in a lysate (QIAzol(R), QIAGEN), and then the cells were crushed using a crushing device (Tissue Lyser, QIAGEN) to obtain a crushed solution. RNA was purified from the crushed solution using an RNA purification kit (miRNeasy Mini Kit(R), QIAGEN) to collect purified RNA. The collected RNA was sent to Mitsubishi Chemical Co., Ltd., which provided contract analysis services, and gene expression in cells treated with the sample was analyzed using an mRNA expression analysis chip. Gene expression in cells not treated with the sample (control) was normalized to 1.00, and the ratio of gene expression in the cells treated with the sample to gene expression in control was calculated. A significance difference test was also performed using a Student t test.

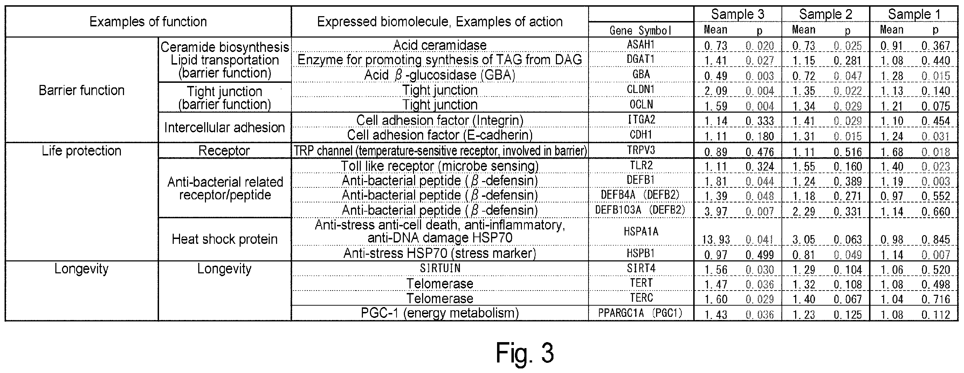

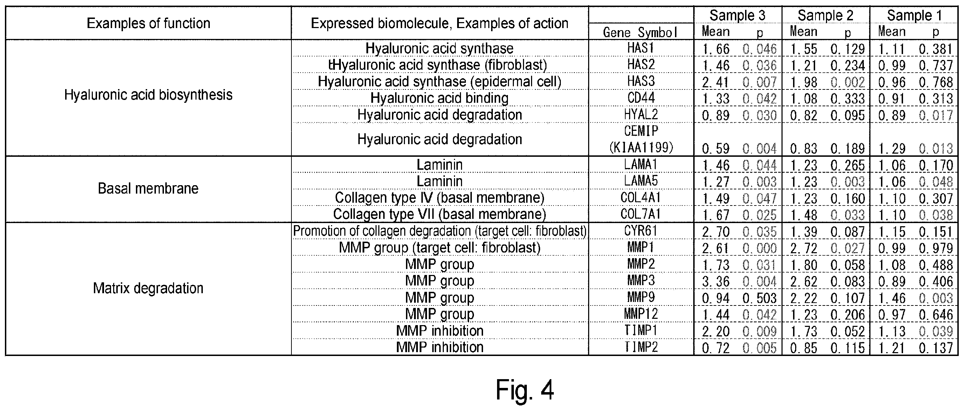

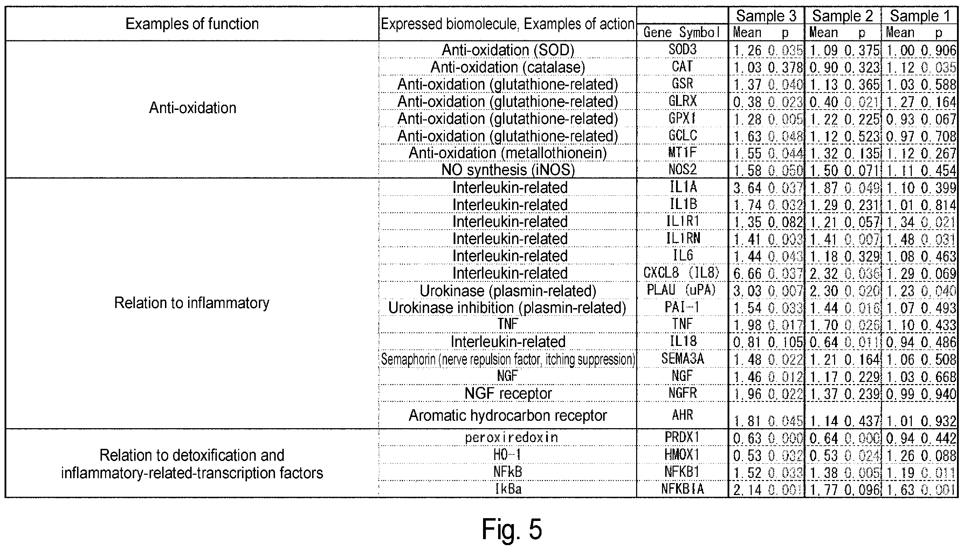

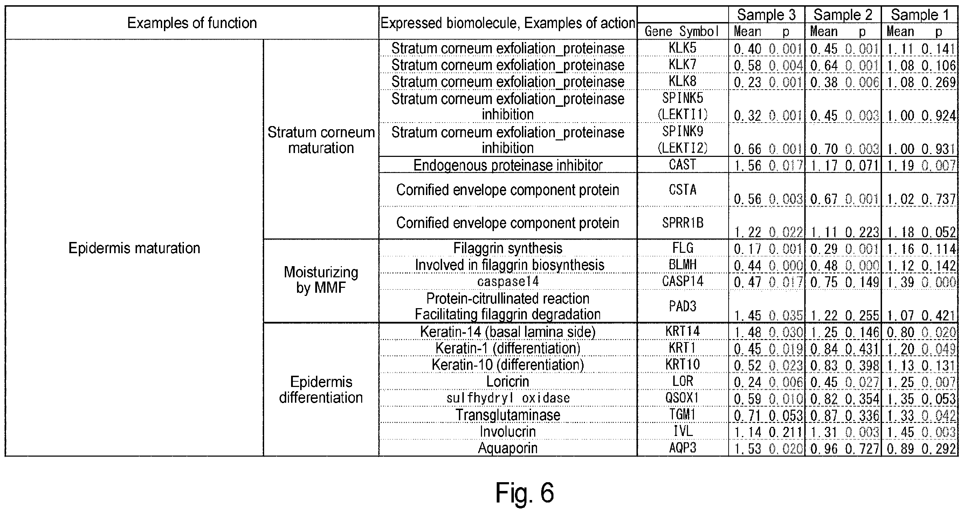

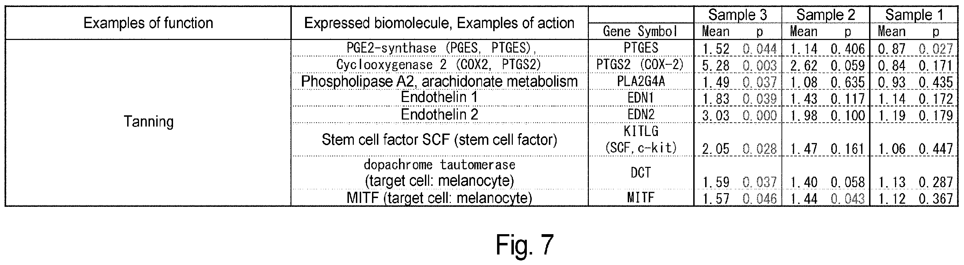

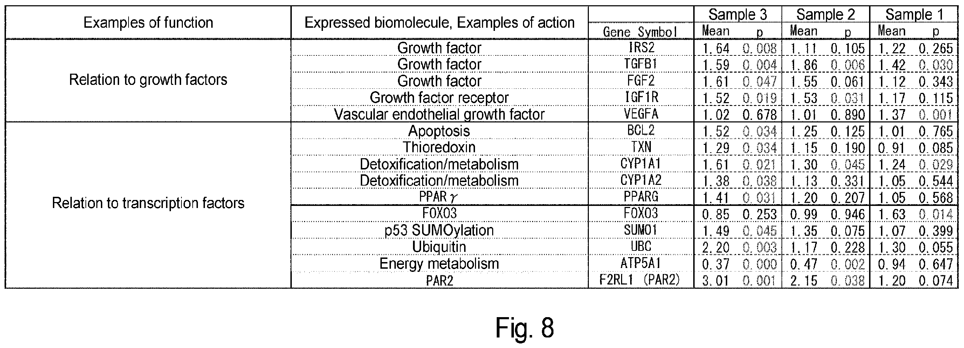

The results are shown in Figures 3 to 8. A value greater than 1.00 indicates that gene expression was promoted than control. A value smaller than 1.00 indicates that gene expression was suppressed than control.

### (Example 5: Anti-wrinkle effect)

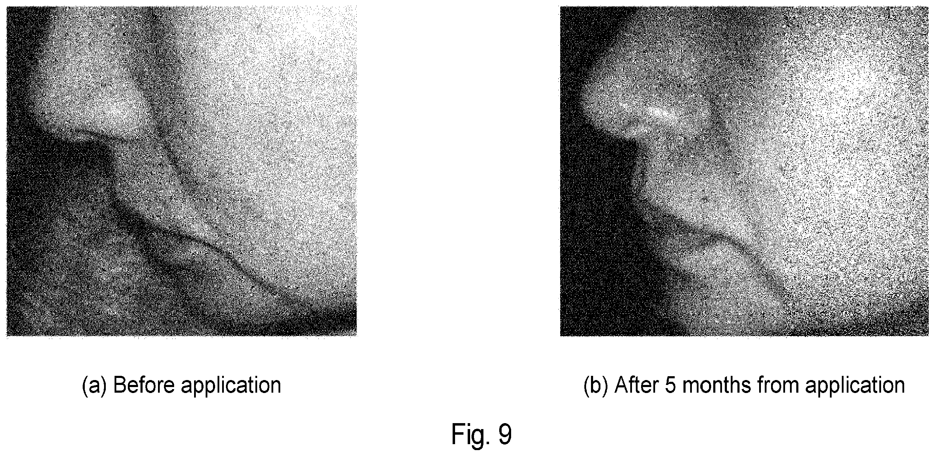

A fermentation liquid of *Artemisia indica var. maximowiczii* containing the *Lactobacillus* sp. prepared in Example 1 at a concentration of 5.0 g/L was applied to the cheek of a 46-year-old woman twice daily for 5 months. As a result, it was confirmed that wrinkles with aging were reduced as shown in Figure 9. It should be noted that no inflammatory reaction, tanning and the like were confirmed. The fermentation liquid of *Artemisia indica var. maximowiczii* containing the *Lactobacillus* sp. prepared in Example 1 at a concentration of 5.0 g/L was extremely effective in promoting expression of the HSP70 gene as shown in Example 4. Thus, it is believed that the anti-inflammatory and whitening effects of HSP70 exceeded the effects of promoting expression of inflammation-related and tanning-related genes.

### (Example 6: Anti-bacterial effect of Lactobacillus sp.)

Staphylococcus aureus and MRSA were prepared as gram-positive cocci. Bacillus subtilis and Bacillus cereus were prepared as gram-positive rod. Escherichia coli, Salmonella enterica, Vibrio enteritidis, and Bacillus pneumoniae were prepared as gram-negative cocci. Pseudomonas aeruginosa was prepared as gram-negative rod.

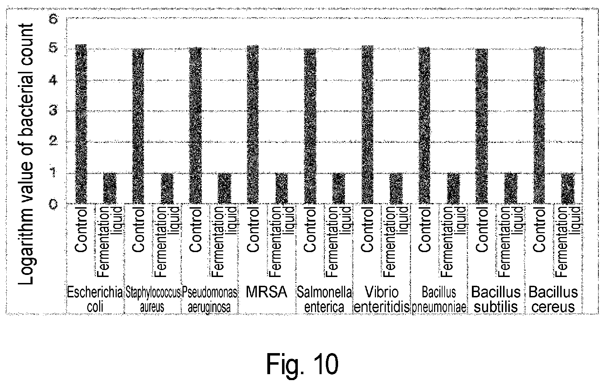

To the soy milk fermentation liquid containing 10 mL of the *Lactobacillus* sp. prepared in Example 3, 0.1 mL of a bacterial solution containing any one of the above bacteria at a concentration of 10⁷ cells/mL was inoculated and allowed to react at 25°C, and the bacterial viability of the inoculated bacteria over time was measured for 24 hours. Also, as a control, 0.1 mL of the bacterial solution was inoculated into 10 mL of 1/15 mol/L phosphate buffer of pH 7.2, and allowed to react at 25°C, and the bacterial viability of the inoculated bacteria over time was measured for 24 hours. As a result, as shown in Figure 10, the soy milk fermentation liquid containing the *Lactobacillus* sp. reduced all types of the prepared bacteria within 24 hours.

### (Example 7: Anti-fungal effect of Lactobacillus sp.)

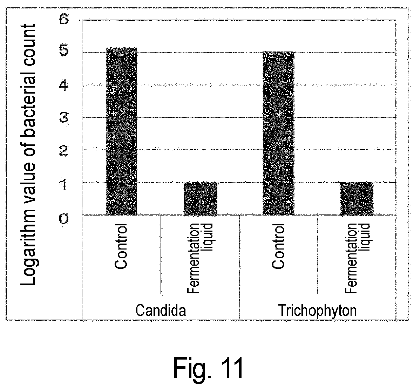

Trichophyton and Candida were prepared as fungi. To the soy milk fermentation liquid containing 10 mL of the *Lactobacillus* sp. prepared in Example 3, 0.1 mL of a bacterial solution containing Trichophyton or Candida at a concentration of 10⁷ cells/mL was inoculated and allowed to react at 25°C, and the bacterial viability of the inoculated bacteria over time was measured for 24 hours. Also, as a control, 0.1 mL of the bacterial solution was inoculated into 10 mL of 1/15 mol/L phosphate buffer of pH 7.2, and allowed to react at 25°C, and the bacterial viability of the inoculated bacteria over time was measured for 24 hours. As a result, as shown in Figure 11, the soy milk fermentation liquid containing the *Lactobacillus* sp. reduced the prepared Trichophyton and Candida within 24 hours.

### (Example 8: Anti-viral effect of Lactobacillus sp.)

A culture solution of influenza virus type A (H1N1) was prepared as an envelope virus. In addition, a culture solution of norovirus (feline calicivirus) was prepared as a non-envelope virus. The culture solution of virus was serially diluted by 10-fold with purified water. An anti-viral test with the soy milk fermentation liquid containing the *Lactobacillus* sp. prepared in Example 3 was then performed at room temperature according to 50% tissue culture infectious dose (TCID 50). The anti-viral test was conducted at the Japan Food Research Laboratories.

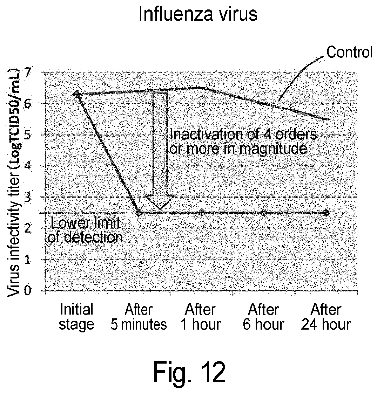

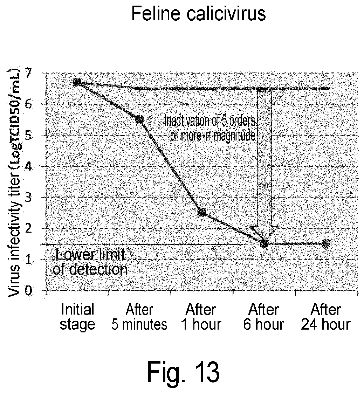

As a result, the soy milk fermentation liquid containing the *Lactobacillus* sp. reduced the infectious titer of influenza virus within 1 hour as shown Figure 12. Also, the soy milk fermentation liquid containing the *Lactobacillus* sp. reduced the infectious titer of norovirus within 24 hours as shown Figure 13.

### (Example 9: Anti-Trichophyton effect of Lactobacillus sp.)

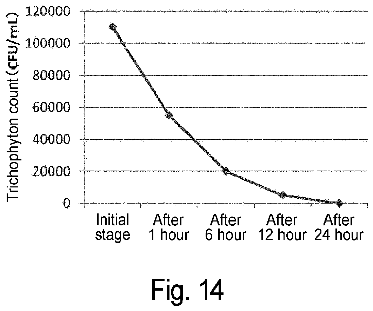

The non-heat-treated fermentation liquid of *Artemisia indica var. maximowiczii* obtained in Example 1 was spray-dried to obtain dried bacterial cells of the *Lactobacillus* sp. The obtained dried bacterial product was suspended in water and glycerin so that the dried bacterial product was 10 parts by weight to obtain a suspension of the *Lactobacillus* sp. of Example 9. The suspension of the *Lactobacillus* sp. was added to Trichophyton, and the colony-forming unit (CFU) of Trichophyton was measured. As a result, the suspension of the *Lactobacillus* sp. killed Trichophyton within 24 hours as shown in Figure 14.

### (Example 10: Collagen type I and HSP47 production promoting effect of Lactobacillus sp.)

Normal human dermal fibroblasts were maintained using a DMEM medium (+5% FBS) in an incubator until confluent. After reaching confluence, the medium was removed from the incubator. DMEM (0% FBS) containing 600 µmol/L of hydrogen peroxide (H₂O₂) was then added to the incubator, and the cells were cultured at 37°C for 1 hour. After 1 hour of culturing, the H₂O₂-containing DMEM (0% FBS) was removed from the incubator, and then a DMEM medium (+10% FBS) was added to the incubator. After these procedures were repeated for 4 days, the cells were cultured with DMEM (10% FBS) for an additional 3 days, and the obtained cells were used as aging induction-treated cells.

The aging induction of the cells was confirmed by staining with senescence-associated beta-galactosidase (SA-β-Gal), an aging marker.

The aging induction-treated cells were seeded in a 48-hole plate at a cell density of 5.0 x 10⁴ cells/well using a DMEM medium (+5% FBS). Twenty-four hours after seeding, the medium was replaced with each of a DMEM medium (+0.5% FBS) containing the non-heat-treated fermentation liquid of *Artemisia indica var. maximowiczii* prepared in Example 1 at concentrations of 1.0% and 10.0%, a DMEM medium (+0.5% FBS) containing the non-heat-treated fermentation liquid of *Angelica keiskei* prepared in Example 2 at concentrations of 1.0% and 10.0%, a DMEM medium (+0.5% FBS) containing Vitamin C magnesium phosphate at 25 µmol/L, a DMEM medium (+0.5% FBS) containing Vitamin C at 25 µmol/L, and a DMEM medium (+0.5% FBS). The cells were then cultured for 48 hours, and then the medium was collected. The cells were washed with PBS(-), then trypsinized, and were collected from the plate. The collected cells were sonicated, and the resulting cell lysate was centrifuged at 15000 rpm to collect the supernatant.

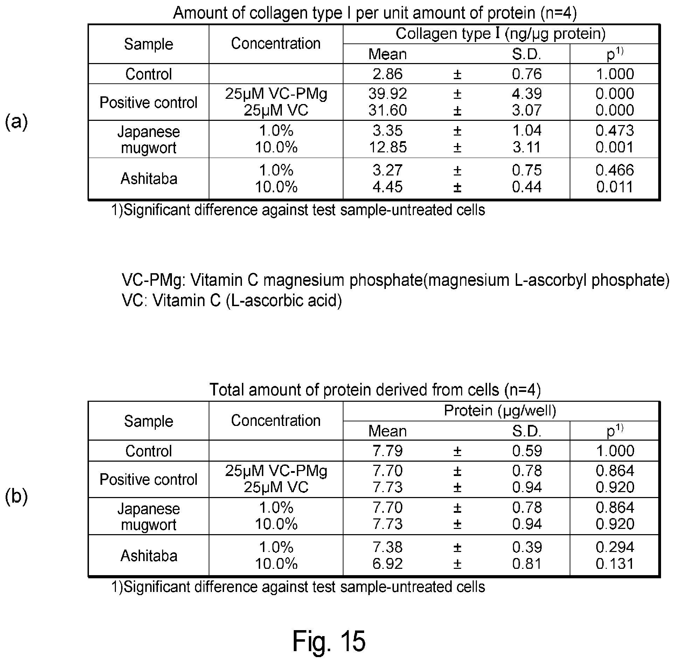

The amount of collagen type I in the collected medium was quantified by an ELISA method (direct method using anti-human collagen type I antibody (rabbit)). As a result, as shown in Figure 15, it was shown that culturing cells in a medium containing Vitamin C magnesium phosphate or Vitamin C (positive control) promoted production of collagen type I. Furthermore, it was shown that the production of collagen type I was also promoted when cells were cultured in each of the medium to which the fermentation liquid of *Artemisia indica var. maximowiczii* was added and the medium to which the fermentation liquid of *Angelica keiskei* was added.

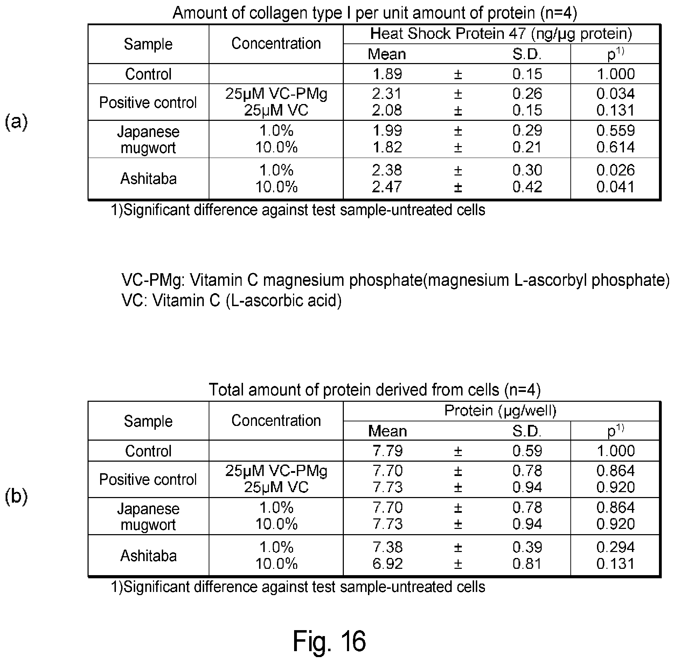

In addition, HSP47 in the supernatant of the cell lysate was quantified using a commercially available ELISA kit (abcam). As a result, it was shown that culturing cells in a medium to which the fermentation liquid of *Angelica keiskei* was added promoted production of HSP47 as shown in Figure 16.

### (Example 11: Anti-aging effect of Lactobacillus sp.)

Normal human dermal fibroblasts were seeded in a six-hole plate at a cell density of 5.0 x 10⁵ cells/well using a DMEM medium (+5% FBS), and the cells were cultured for 24 hours.

The medium of each well was removed. DMEM (0% FBS) containing 600 µmol/L of hydrogen peroxide (H₂O₂) was then added to the well, and the cells were cultured at 37°C for 1 hour to induce aging of the cells. After 1 hour of culture, the H₂O₂-containing DMEM (0% FBS) was removed from the wells, then each of a DMEM medium (+10% FBS) containing 1.0% of the fermentation liquid of *Artemisia indica var. maximowiczii* containing the *Lactobacillus* sp. prepared in Example 1 at a concentration of 5.0 g/L, a DMEM medium (+10% FBS) containing 1.0% of the fermentation liquid of *Angelica keiskei* containing the *Lactobacillus* sp. prepared in Example 2 at a concentration of 5.0 g/L, a DMEM medium (+10% FBS) containing 10 µmol/L of resveratrol, which has an anti-oxidation effect, as a positive control, and a DMEM medium (+10% FBS) as a negative control was added to the wells. These procedures were repeated for 4 days.

Subsequently, each of a DMEM medium (+10% FBS) containing 1.0% of the fermentation liquid of *Artemisia indica var. maximowiczii* containing the *Lactobacillus* sp. prepared in Example 1 at a concentration of 5.0 g/L, a DMEM medium (+10% FBS) containing 1.0% of the fermentation liquid of *Angelica keiskei* containing the *Lactobacillus* sp. prepared in Example 2 at a concentration of 5.0 g/L, a DMEM medium (+10% FBS) containing 10 µmol/L of resveratrol, which has an anti-oxidation effect, as a positive control, and a DMEM medium (+10% FBS) as a negative control was added to the wells, and the cells were cultured for 3 days.

The cells cultured under each condition were then seeded in a 48-hole plate at a cell density of 5.0 x 10⁴ cells/well, and the cells were cultured for 24 hours. The cells were then immobilized with PBS containing 3% formaldehyde. The solution in the well was then replaced with a reaction solution at pH 6 containing 1 mg/mL of 5-bromo-4-chloro-3-indolyl-D-galactoside (X-Gal), and the wells were allowed to stand still for 12 to 16 hours. Microscopic observation was then performed, and staining with senescence-associated beta-galactosidase (SA-β-Gal), an aging marker, was observed.

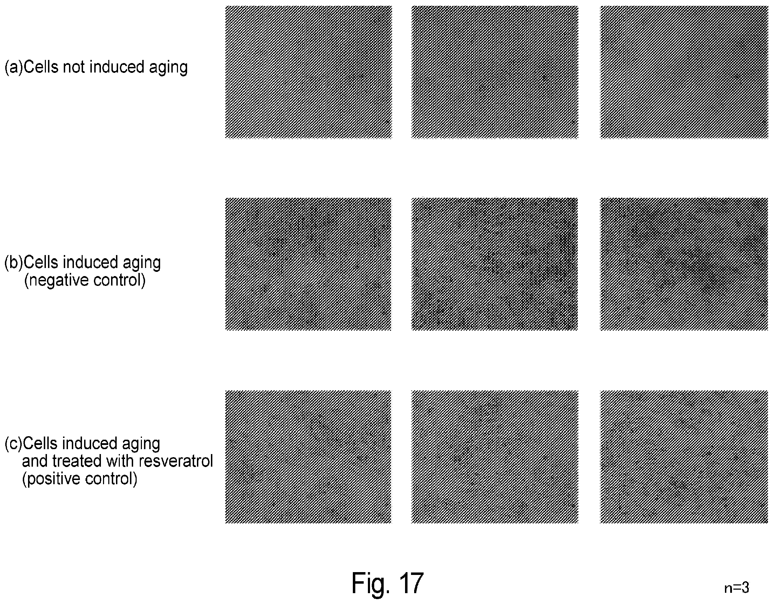

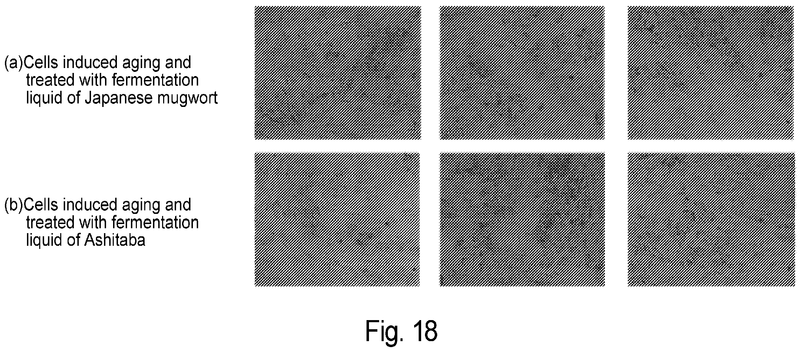

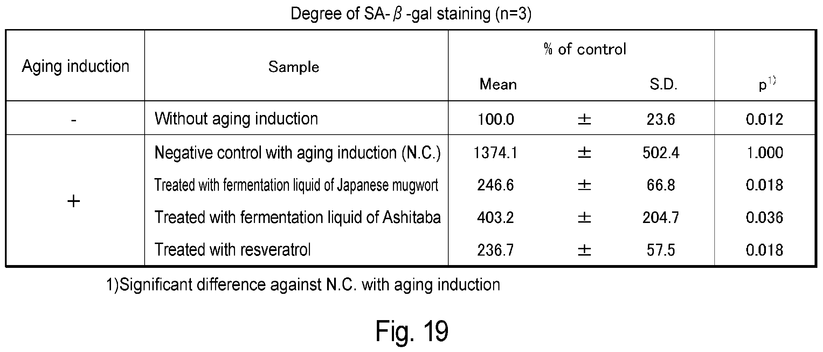

As a result, the cells induced aging alone had a strong degree of SA-β-Gal staining, as shown in Figures 17, 18 and 19. In contrast, the cells induced aging and treated with the fermentation liquid of *Artemisia indica var. maximowiczii* or the fermentation liquid of *Angelica keiskei* reduced the degree of SA-β-Gal staining, as did the cells induced aging and treated with resveratrol. Thus, it was shown that the fermentation liquid of *Artemisia indica var. maximowiczii* and the fermentation liquid of *Angelica keiskei* have an anti-aging effect.

### (Example 12: HSP70 production promoting effect of Lactobacillus sp.)

A three-dimensional culture epidermal model (SkinEthic RHE: 19RHE 008, EPISKIN, Inc.) was conditioned overnight in a growth medium (Growth Medium: 19SGM 005, EPISKIN, Inc.). After conditioning, the epidermal model was transferred to a 6-hole plate dispensed with 1 mL of the growth medium, and each of 50 µL of the fermentation liquid of *Artemisia indica var. maximowiczii* containing the *Lactobacillus* sp. prepared in Example 1 at a concentration of 5.0 g/L and 50 µL of the fermentation liquid of *Angelica keiskei* containing the *Lactobacillus* sp. prepared in Example 2 at a concentration of 5.0 g/L was applied to the stratum corneum side of the epidermal model.

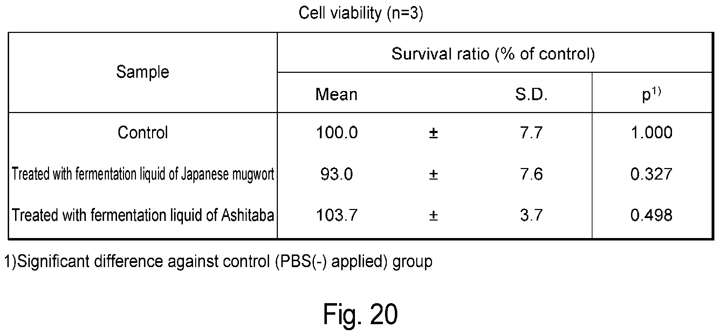

The epidermal model was cultured for 24 hours, and then the fermentation liquid was removed, and the epidermal model was washed with PBS(-). The viability of the epidermal model was assessed using an Alamer Blue method. A maintenance medium containing 10% Alamer Blue reagent (alamarBlue Cell Viability Reagent(R), Invitrogen, Inc.) was dispensed into a 24-hole plate. The epidermal model was transferred to the plate, and cultured for 2 hours, and then the fluorescence intensity of the culture supernatant was measured. The cell viability was calculated as an index (%) to the fluorescence intensity of the control group to which PBS(-) was applied in place of the fermentation liquid. The obtained results were subjected to a significance test using a Student t test. As a result, as shown in Figure 20, there was no significant decrease in the cell viability by the application of the fermentation liquid of *Artemisia indica var. maximowiczii* or the fermentation liquid of *Angelica keiskei.*

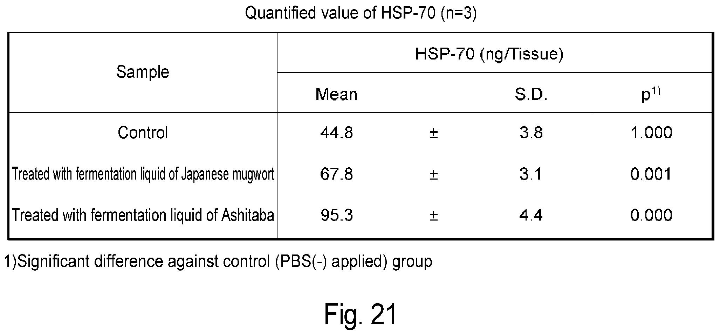

After the fluorescence intensity was measured, the epidermal model was immersed in PBS, and the epidermal model was crushed using a sample crusher (Tissue Lyser). The cell lysate was centrifuged at 15000 rpm, and the supernatant was collected. The HSP70 in the collected solution was quantified using a commercially available ELISA kit (Enzo Life Sciences, Inc.). The obtained results were subjected to a significance test using a Student t test. As a result, the amount of HSP70 produced was significantly increased by the application of the fermentation liquid of *Artemisia indica var. maximowiczii* or the fermentation liquid of *Angelica keiskei* as shown in Figure 21.

The above-described embodiments and examples are for ease of understanding the present invention, and are not for construing the present invention in a limiting manner. The present invention may be modified/improved without departing from its spirit, and the present invention also includes equivalents thereof. In other words, those in which a skilled person in the art has made appropriate design changes to each of the embodiments and examples are also encompassed within the scope of the present invention as long as they have the features of the present invention. For example, the various elements included in the embodiments and the examples are not limited to those illustrated, and can be changed as appropriate. Furthermore, the embodiments and examples each are exemplary, and it goes without saying that partial substitutions or combinations of the configurations shown in different embodiments are possible and those are also encompassed within the scope of the present invention as long as they have the features of the present invention.

## Claims

1. An agent for modulating expression of a heat shock protein gene, comprising a *Lactobacillus* sp. derived from *Artemisia indica var. maximowiczii* or *Angelica keiskei.*

2. The agent for modulating expression of a heat shock protein gene according to claim 1, wherein the heat shock protein gene is at least one selected from the group consisting of an HSPA1A gene and an HSPB1 gene, and the agent promotes expression of at least one selected from the group consisting of the HSPA1A gene and the HSPB1 gene.

3. The agent for modulating expression of a heat shock protein gene according to claim 1 or 2, wherein the agent modulates expression of at least one selected from the group consisting of a ceramidase gene, a lipid synthase gene, a lysosomal hydrolase gene, a tight junction biosynthesis factor gene, and an intercellular adhesion factor gene.

4. The agent for modulating expression of a heat shock protein gene according to claim 3, wherein
the ceramidase gene is an ASAH1 gene and the agent suppresses expression of the ASAH1 gene;
the lipid synthetase gene is a DGAT1 gene and the agent promotes expression of the DGAT1 gene;
the lysosomal hydrolase gene is a GBA gene and the agent promotes expression of the GBA gene;
the tight junction biosynthesis factor gene is at least one selected from the group consisting of a CLDN1 gene and an OCLN gene, and the agent promotes expression of at least one selected from the group consisting of the CLDN1 gene and the OCLN gene; and
the intercellular adhesion factor gene is at least one selected from the group consisting of an ITGA2 gene, a CDH1 gene, and a CD44 gene, and the agent promotes expression of at least one selected from the group consisting of the ITGA2 gene, the CDH1 gene, and the CD44 gene.

5. The agent for modulating expression of a heat shock protein gene according to claim 1 or 2, wherein the agent modulates expression of an anti-microbial molecule gene.

6. The agent for modulating expression of a heat shock protein gene according to claim 5, wherein the anti-microbial molecule gene is at least one selected from the group consisting of a TLR2 gene, a DEFB1 gene, a DEFB4A gene, and a DEFB103A gene, and the agent promotes expression of at least one selected from the group consisting of the TLR2 gene, the DEFB1 gene, the DEFB4A gene, and the DEFB103A gene.

7. The agent for modulating expression of a heat shock protein gene according to claim 1 or 2, wherein the agent modulates expression of at least one selected from the group consisting of a sirtuin gene and a telomerase gene.

8. The agent for modulating expression of a heat shock protein gene according to claim 7, wherein
the sirtuin gene is a SIRT4 gene, and the agent promotes expression of the SIRT4 gene; and
the telomerase gene is at least one selected from the group consisting of a TERT gene and a TERC gene, and the agent promotes expression of at least one selected from the group consisting of the TERT gene and the TERC gene.

9. The agent for modulating expression of a heat shock protein gene according to claim 1 or 2, wherein the agent modulates expression of an energy metabolizing factor gene.

10. The agent for modulating expression of a heat shock protein gene according to claim 9, wherein the energy metabolizing factor gene is a PPARGC1A gene, and the agent promotes expression of the PPARGC1A gene.

11. The agent for modulating expression of a heat shock protein gene according to claim 1 or 2, wherein the agent modulates expression of at least one selected from the group consisting of a hyaluronic acid biosynthesis factor gene and a hyaluronic acid degradation factor gene.

12. The agent for modulating expression of a heat shock protein gene according to claim 11, wherein
the hyaluronic acid biosynthesis factor gene is at least one selected from the group consisting of a HAS1 gene, a HAS2 gene, and a HAS3 gene, and the agent promotes expression of at least one selected from the group consisting of the HAS1 gene, the HAS2 gene, and the HAS3 gene; and
the hyaluronic acid degradation factor gene is at least one selected from the group consisting of a HYAL2 gene and a CEMIP gene, and the agent suppresses expression of at least one selected from the group consisting of the HYAL2 gene and the CEMIP gene.

13. The agent for modulating expression of a heat shock protein gene according to claim 1 or 2, wherein the agent modulates expression of a basal membrane biosynthesis factor gene.

14. The agent for modulating expression of a heat shock protein gene according to claim 13, wherein the basal membrane biosynthesis factor gene is at least one selected from the group consisting of a LAMA1 gene, a LAMA5 gene, a COL4A1 gene, and a COL7A1 gene, and the agent promotes expression of at least one selected from the group consisting of the LAMA1 gene, the LAMA5 gene, the COL4A1 gene, and the COL7A1 gene.

15. The agent for modulating expression of a heat shock protein gene according to claim 1 or 2, wherein the agent promotes production of collagen type I.

16. The agent for modulating expression of a heat shock protein gene according to claim 1 or 2, wherein the agent comprises a *Lactobacillus* sp. derived from the *Angelica keiskei,* and the agent promotes production of heat shock protein 47.

17. The agent for modulating expression of a heat shock protein gene according to any one of claims 1 to 16, wherein the agent further comprises a fermentation liquid derived from the *Artemisia indica var. maximowiczii* or *Angelica keiskei.*

18. The agent for modulating expression of a heat shock protein gene according to any one of claims 1 to 17, wherein the *Lactobacillus* sp. is at least one selected from the group consisting of *L. parafarraginis, L. parabuchneri, L. buchneri, L. harbinensis, L. vini,* and *L. nagelii.*

19. The agent for modulating expression of a heat shock protein gene according to any one of claims 1 to 18, wherein the *Lactobacillus* sp. is a dead bacterium.

20. The agent for modulating expression of a heat shock protein gene according to any one of claims 1 to 19, wherein the *Lactobacillus* sp. is heat-treated.

21. The agent for modulating expression of a heat shock protein gene according to any one of claims 1 to 20, wherein the *Lactobacillus* sp. is a dried bacterial product.

22. A medicament comprising the agent for modulating expression of a heat shock protein gene according to any one of claims 1 to 21.

23. The medicament according to claim 22, wherein the medicament is an anti-wrinkle medicament.

24. The medicament according to claim 22, wherein the medicament is an anti-aging medicament.

25. A cosmetic comprising the agent for modulating expression of a heat shock protein gene according to any one of claims 1 to 21.

26. The cosmetic according to claim 25, wherein the cosmetic is an anti-wrinkle cosmetic.

27. The cosmetic according to claim 25, wherein the cosmetic is an anti-aging cosmetic.

28. A method for manufacturing an agent for modulating expression of a heat shock protein gene, comprising obtaining a *Lactobacillus* sp. from *Artemisia indica var. maximowiczii* or *Angelica keiskei.*

29. The method for manufacturing an agent for modulating expression of a heat shock protein gene according to claim 28, further comprising fermenting the *Artemisia indica var. maximowiczii* or *Angelica keiskei* to obtain a fermentation liquid.
